# EUROPEAN PATENT APPLICATION

(11) **EP 0 812 598 A2**
(43) Date of publication of application: **17.12.1997**
(21) Application number: 97105716.1
(22) Date of filing: 07.04.1997
(51) Int. Cl.: A61M 11/00

(54) **Fluid-tight aerosol apparatus**

(30) Priority: 12.06.1996 IT MI961192
(71) Applicant: MIAT S.P.A., I-20159 Milan (IT); MED 2000 S.r.l, 25010 Pozzolengo (Brescia) (IT)
(72) Inventor: Fraccaroli, Nicola, 25015 Desenzano Del Garda, Brescia (IT); Fraccaroli, Davide, 25010 Sirmione - Brescia (IT)
(74) Representative: Luksch, Giorgio, Dr.-Ing.

(57) **Abstract**

An ultrasonic aerosol apparatus, of the type comprising a hollow casing (1) presenting in its interior a seat (2) for housing at least electrical means (12) generating ultrasound for atomizing a substance to be inhaled, and means (14) for powering said electrical means (12), in which the casing (1) is fluid-tight.

## Description

This invention relates to an ultrasonic aerosol apparatus, in accordance with the precharacterising part of the main claim.

Apparatus of the aforesaid known type all have a serious drawback relating to the possibility of water or other liquid infiltration into the interior of the apparatus casing, in which the electronic components of the apparatus are housed.

As is well known, an aerosol apparatus must always be carefully cleaned after use for hygiene reasons. However, it often happens that because of handling errors by the user, wash liquid or medicinal residues penetrate into the casing housing the electrical components of the apparatus. Such infiltration results in oxidation of the contacts and/or of the printed circuits and/or of the electronic components of the apparatus, this often resulting in malfunction or breakage of the apparatus.

Moreover, on drying, the infiltrations often generate agglomerates which compromise the proper operation of the apparatus.

In particular, it has been found that in known apparatus, liquid infiltration often occurs at the power cable connection socket and/or at the pushbutton for switching the apparatus on and off.

In this respect, even if these components are fixed to the apparatus casing via an interposed gasket, over the long term their presence results in the appearance of infiltration into the apparatus casing housing the electronic components. This happens both because of the degradation to which the gaskets are subject, and because the frequent user-contact with these components loosens them from the apparatus casing.

An object of the present invention is to provide an aerosol apparatus having a casing housing the apparatus electronic components which ensures a perfect seal.

A further object is to provide an aerosol apparatus in which the control and electrical powering means for the apparatus are connected to the outside indirectly, so as to eliminate apertures through which wash water or medicinal residues can infiltrate.

These and further objects which will be apparent to an expert of the art are attained by an aerosol apparatus in accordance with the characterising part of the main claim.

The present invention will be more apparent from the accompanying figures which are provided by way of example and in which equal numerals indicate identical or equivalent parts.

In the figures:
Figure 1 is a schematic cross-section through an apparatus of the invention;
Figure 2 is a circuit diagram of the electronic components provided within the apparatus.

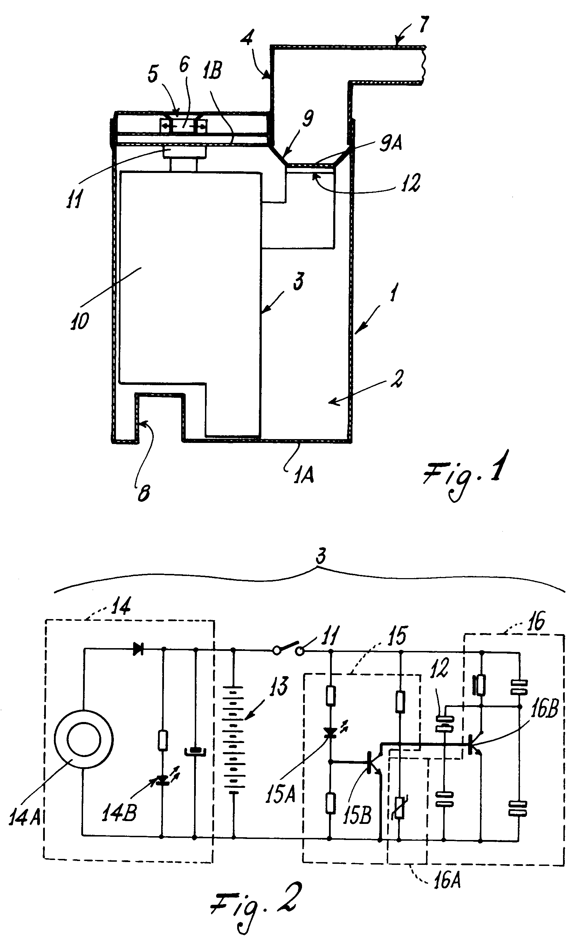

With reference to said figures, an aerosol apparatus according to the invention comprises a first hollow casing 1 for housing the electronic components 3 of the apparatus, and a second casing 4 to be removably connected to the upper end 1B of the first casing in any manner conventional to the expert of the art. The first casing 1 comprises advantageously two hollow shells (only one is shown in Figure 1) sealedly joined together along their perimetral edges, for example by gluing or electric welding, and defining a seat 2 to house the apparatus electronic components, indicated overall by 3.

The casing 1 is of substantially parallelepiped form and in its lower end comprises a recessed seat 8 able to house an external recharging coil (not shown) as described hereinafter and, in its upper end 1B a "cup-like" recessed seat 9 to contain the medicament to be atomized. In its interior the casing 1 comprises usual support elements (not shown but of conventional type), to which there are secured the apparatus electronic components, and in particular a printed circuit 10, a reed switch 11 and a piezoelectric ceramic 12. It should be noted that the switch 11 is positioned within the casing 1, in correspondence with a seat 5 in the other apparatus casing 4, which houses a magnetic element, for example a permanent magnet, arranged to operate the switch 11. The piezoelectric ceramic 12 is positioned at the base of the cup-like seat 9.

The apparatus upper casing 4 comprises the said seat 5 for the magnetic element and, in correspondence with the cup seat 9, a mouthpiece 7 of conventional shape to allow the medicament to be inhaled.

With reference to Figure 2, the apparatus comprises: a conventional rechargeable battery 13 to power the piezoelectric ceramic 12, a battery recharging circuit 14, the switch 11, a usual circuit 15 for intermittently powering the ceramic 12, and a usual oscillator circuit 16, 16A which besides generating in the ceramic 12 the desired resonance frequency and hence the desired vibration, also prevents the temperature of the ceramic 12 exceeding a predetermined value.

The electrical feed is provided to the aerosol apparatus by magnetic induction. For this purpose, the recharging circuit 14 for the battery 13 comprises a secondary winding 14A across which the voltage required for powering the oscillator 16 is induced. This occurs by virtue of a primary winding, external to the apparatus, which induces the necessary voltage in the secondary 14A, and the relative electrical circuit. This latter and the primary winding are of conventional type to the expert of the art and are therefore not described in detail. It should however be noted that, advantageously, the primary winding is shaped in such a manner as to be able to be at least partially inserted into the seat 8 in the casing 1 of the apparatus, and that the secondary winding 14A is wound inside the casing 1 in correspondence with the wall bounding the seat 8.

The circuit 14 also advantageously comprises a light emitting diode 14B which indicates when the apparatus is being recharged.

The battery 13 is a usual rechargeable battery.

As already stated, the switch 11 is a reed switch and is arranged to close or open the connection between the battery 13 and the oscillator 16 connected to the ceramic 12, depending on the position assumed by the magnetic element 6.

The circuit 15 is also of conventional type and will therefore not be described in detail. It comprises an intermittent light emitting diode 15A which, being associated with a transistor 15B, behaves substantially as an ON-OFF oscillator. It should be noted that by virtue of the intermittent feed circuit 15 for the ceramic 12, the life of the battery 13 is prolonged.

For controlling the temperature of the ceramic 12, the oscillating circuit 16, 16A advantageously comprises an impedance component 16A of negative temperature coefficient, or NTC component. It should be noted that the NTC component is positioned in correspondence with or in direct contact with the ceramic 12, and that as the temperature of the ceramic 12 increases, the potential across the NTC component and hence at the base of the transistor 16B decreases, with consequent gradual reduction in the voltage fed by the oscillating circuit, whereas when the temperature decreases the voltage provided by this circuit gradually increases.

Because of the reed switch 11 and the magnetic induction feed circuit of the oscillator 16, the electronic components provided within the apparatus are connected to the outside only indirectly, consequently the casing 1 enclosing the apparatus electronic components can be formed without apertures, so ensuring perfect and permanent impermeability both towards the wash water for the apparatus and towards any medicament residues present in the cup 9.

Finally, it should be noted that the aforegoing embodiment is provided by way of example only, and that numerous modifications are possible all falling within the same inventive concept. For example, to improve ultrasound transmission from the ceramic 12 to the medicament present in the cup seat 9, this latter could comprise the piezoelectric ceramic itself as its end wall, in which case it would be secured to the lower edge of the seat 9 in any known manner ensuring a perfect seal.

A further modification could relate to the switch 12, which could be different from that illustrated provided it can be indirectly operated. A further modification could relate to the apparatus power circuit. In this respect the battery 13 could also be dispensed with. In this case the apparatus would operate by the voltage induced in the winding 14A, in which case during its operation the primary winding would have to be permanently inserted into the seat 8. In this latter embodiment the switch 11 could be dispensed with, and a switch connected into the feed line to the primary winding.

## Claims

1. An ultrasonic aerosol apparatus, of the type comprising a hollow casing (1) presenting in its interior a seat (2) for housing at least electrical means (12) generating ultrasound for atomizing a substance to be inhaled, and means (14) for powering said electrical means (12), characterised in that said casing (1) is fluid-tight.

2. An apparatus as claimed in claim 1, characterised in that said powering means (14) are connected indirectly to the outside of the casing.

3. An apparatus as claimed in claim 2, characterised in that said powering means (14) are of the magnetic induction type.

4. An apparatus as claimed in claim 3, characterised in that said powering means comprise a rechargeable battery (13).

5. An apparatus as claimed in claim 1, characterised in that said powering means comprise inside said casing a secondary winding (14A), and outside said casing (1) a primary winding arranged to induce in the secondary the power required for operating the apparatus.

6. An apparatus as claimed in claim 1, characterised in that the casing is a closed casing free of apertures leading to the outside.

7. An apparatus as claimed in claim 1, characterised in that the casing is a closed casing having an aperture closed in a fluid-tight manner by said electrical means (12).

8. An apparatus as claimed in claim 1, characterised in that the casing (1) comprises within its seat (2) control means (11) for the electrical means (12), said control means being operable indirectly from outside said casing (1).

9. An apparatus as claimed in claim 8, characterised in that the control means (11) comprise a reed switch (11).

10. An apparatus as claimed in claim 1, characterised in that the casing (1) comprises within its seat (2) means (15) for intermittently powering said electrical means (12).
